# FASCICULE DE BREVET EUROPEEN

(11) **EP 1 109 515 B1**
(45) Date de publication et mention de la délivrance du brevet: **22.12.2004**
(21) Numéro de dépôt: 99941690.2
(22) Date de dépôt: 03.09.1999
(51) Int. Cl.: A61F 2/44

(54) **IMPLANT DE TYPE CAGE INTERSOMATIQUE, NOTAMMENT POUR VERTEBRES CERVICALES**
ZWISCHENKÖRPERLICHES WIRBELKÄFIGIMPLANTAT
INTERBODY CAVITY IMPLANT, IN PARTICULAR FOR CERVICAL VERTEBRAE

(30) Priorité: 04.09.1998 FR 9811175
(43) Date de publication de la demande: 27.06.2001
(73) Titulaire: Dimso (Distribution Medicale du Sud-Ouest), 33610 Cestas (FR)
(72) Inventeur: BACCELLI, Christian, F-33640 Ayguemorte les Graves (FR); CONCHY, Frédéric, F-33650 Saint Médard d'Eyrans (FR)
(74) Mandataire: Le Forestier, Eric
(86) Numéro de dépôt international: PCT/FR1999/002107
(87) Numéro de publication internationale: WO 2000/013618

(56) Documents cités:
- EP-A- 0 697 200
- FR-A- 2 730 159
- US-A- 5 236 460
- US-A- 5 609 635
- US-A- 5 800 550

## Description

La présente invention a trait d'une façon générale aux cages intersomatiques destinées à venir en remplacement partiel ou total d'un disque intervertébral dans le traitement chirurgical du rachis et à permettre une fusion osseuse entre les plateaux vertébraux sus- et sous-jacents.

On connaît déjà dans l'état de la technique un certain nombre de types de cages intersomatiques. Une cage particulièrement répandue est constituée par un corps d'un seul tenant en matériau biocompatible tel qu'un alliage de titane ou un acier inoxydable, dont la surface supérieure et la surface inférieure présentent des reliefs tels que des stries de section triangulaire destinées à réaliser un ancrage dans les plateaux vertébraux sus- et sous-jacents lorsque la distraction entre les vertèbres effectuée pour la pose de l'implant est relâchée. Une telle cage est également munie d'au moins une ouverture traversant le corps de haut en bas et débouchant sur ses faces supérieure et inférieure. La cavité que constitue cette ouverture est remplie d'un matériau ostéogène ou de greffon osseux, de manière à assurer à travers celle-ci une croissance osseuse entre les plateaux vertébraux et, à terme, une fusion osseuse rigide entre ces plateaux.

Un implant selon le preambule de la revendication 1 est connue du document EP-A-0 697 200.

Ces cages connues présentent toutefois certaines limitations, notamment en termes de tenue dans le temps de l'ancrage dans les plateaux vertébraux, qui est essentielle pour réaliser la fusion osseuse.

Ces cages sont également mal adaptées à la forme des vertèbres cervicales, dont la configuration concave des plateaux vertébraux peut rendre le principe d'un tel ancrage par stries saillantes insuffisant en soi.

La présente invention vise à pallier ces limitations de l'état de la technique et à proposer un nouvel implant de type cage intersomatique qui soit bien adapté à une implantation dans la région des vertèbres cervicales, et qui soit apte à se solidariser aux plateaux vertébraux avec une excellente qualité d'ancrage.

Un autre objet de l'invention est de proposer un implant de type cage intersomatique dont la pose soit particulièrement simple et dont le poids puisse être diminué par rapport aux cages entièrement métalliques de l'art antérieur, et donc la fabrication puisse être plus simple et moins couteuse.

Ainsi l'invention propose un implant de type cage intersomatique, notamment pour vertèbres cervicales, comprenant un corps possédant une face supérieure et une face inférieure aptes à s'appuyer sur les plateaux vertébraux sus- et sous-jacents et possédant en outre au moins une ouverture traversante débouchant sur lesdites faces supérieure et inférieure, caractérisé en ce qu'il comprend en outre un organe d'ancrage monté de façon à pouvoir se déplacer dans ladite ouverture, ledit organe d'ancrage comprenant une partie d'appui adjacente à un premier plateau vertébral, et au moins un élément saillant d'ancrage s'étendant à partir de ladite partie d'appui en direction du plateau vertébral opposé, de telle sorte que lorsque l'implant est comprimé entre les plateaux vertébraux, ledit organe d'ancrage soit sollicité par le premier plateau vertébral au niveau de ladite partie d'appui pour se déplacer par rapport au corps et pour que ledit élément saillant au moins prévu déborde de la face du corps tournée vers le plateau vertébral opposé et s'ancre dans ce dernier.

Des aspects préférés, mais non limitatifs, de l'implant selon l'invention sont les suivants :
- le corps possède sur au moins l'une de ses faces supérieure et inférieure des aménagements d'ancrage complémentaire.
- ledit organe d'ancrage possède une pluralité d'éléments saillants.
- lesdits éléments saillants sont allongés et essentiellement parallèles entre eux.
- le ou chaque élément saillant d'ancrage présente la forme d'une tige à extrémité libre en pointe.
- ladite ouverture du corps possède un aménagement de butée pour ledit organe d'ancrage, afin de limiter la course du déplacement de celui-ci provoqué par ledit premier plateau vertébral.
- ledit aménagement est constitué par un épaulement entre deux parties de tailles différentes de ladite ouverture.
- ladite partie d'appui de l'organe d'ancrage s'adapte étroitement dans la partie de plus grande taille de ladite ouverture.
- lorsque l'organe d'ancrage s'appuie contre l'aménagement de butée, la partie d'appui dudit organe est entièrement inscrite dans la face inférieure du corps.
- l'organe d'ancrage possède une pluralité d'éléments saillants d'ancrage s'adaptant ensemble étroitement dans la partie de plus petite taille de ladite ouverture.
- ladite partie d'appui de l'organe d'ancrage présente la forme d'un anneau dont une ouverture centrale assure la continuité du passage entre les faces supérieure et inférieure du corps à travers ladite ouverture du corps.
- le corps présente une forme extérieure généralement arrondie, avec des zones de forte courbure et des zones de courbure moindre.
- ladite ouverture du corps présente un contour de forme semblable à ladite forme extérieure du corps.
- il est prévu sur l'organe d'ancrage une pluralité d'éléments saillants situés au voisinage de zones de forte courbure de ladite ouverture et reçus dans des renfoncements formés dans ces zones.
- l'une des faces supérieure et inférieure du corps est convexe.
- la partie d'appui de l'organe d'ancrage est généralement plane.
- le corps est réalisé en un matériau polymère biocompatible tel qu'un polyéther-éther-cétone.
- l'organe d'ancrage est réalisé en alliage métallique biocompatible.

D'autres aspects, buts et avantages de la présente invention apparaîtront mieux à la lecture de la description détaillée suivante d'une forme de réalisation préférée de celle-ci, donnée à titre d'exemple non limitatif et faite en référence aux dessins annexés, sur lesquels:
la figure 1 est une vue en perspective d'un implant selon l'invention,
la figure 2 est une vue en élévation de face de l'implant de la figure 1,
la figure 3 est une vue de dessous de l'implant des figures 1 et 2,
la figure 4 est une vue de dessus de l'implant des figures 1 à 3,
la figure 5 est une vue en coupe longitudinale selon la ligne V-V de la figure 4,
la figure 6 est une vue en coupe transversale selon la ligne VI-VI de la figure 4, dans une première position de l'implant, et
la figure 7 est une vue analogue à la figure 6, dans une seconde position de l'implant.

En référence maintenant au dessin, on a représenté un implant destiné à former une cage intersomatique entre deux plateaux vertébraux, de préférence au niveau des vertèbres cervicales, qui est constitué de deux parties, à savoir un corps de cage extérieur 10 et une armature intérieure 20.

Le corps de cage présente en l'espèce, en vue de dessus, un contour généralement triangulaire à coins arrondis. D'autres contours, de préférence tous à coins arrondis, peuvent également être adoptés

Le corps 10 possède une face supérieure généralement plane 14 munie de stries 141 de section triangulaire, s'étendant dans une direction essentiellement perpendiculaire à la direction de mise en place entre les plateaux vertébraux et destinées à participer à l'ancrage dans le plateau vertébral sus-jacent.

La face inférieure 13 du corps présente quant à elle une convexité adaptée pour être essentiellement complémentaire de la concavité naturelle de la face supérieure du plateau vertébral cervical sous-jacent. Cette face inférieure 13 présente également des stries 131 réalisant l'ancrage dans ledit plateau vertébral sous-jacent.

Entre ces faces supérieure et inférieure s'étend une face périphérique 12 généralement lisse, dans une région antérieure de laquelle est formée une ouverture 121, de préférence munie d'un taraudage, ceci pour permettre lors de la pose de monter l'implant à l'extrémité d'un instrument, de type classique en soi.

Le corps 10 est traversé par une cavité 11 débouchant sur sa face supérieure et sur sa face inférieure, cette cavité étant constituée par une partie supérieure 111 et une partie inférieure 112 légèrement plus large que la partie supérieure, de manière à définir à la transition entre ces parties un épaulement 113, à des fins expliquées plus loin.

On observe que la paroi 16 définissant la cavité 11 adopte la même forme ici généralement triangulaire à coins arrondis que le contour extérieur du corps 10.

L'armature 20 est constituée par une pièce conçue pour pouvoir être reçue à l'intérieur de la cavité 11. Cette armature présente à sa base un anneau 22 s'étendant dans un plan général horizontal et dont le contour extérieur épouse sensiblement, sur au moins une partie substantielle de son étendue, la forme de la paroi de la partie basse 112 de la cavité 11 du corps 10, de manière à pouvoir être guidé en translation verticale dans ladite partie basse. Cet anneau est traversé par une ouverture centrale 221.

A partir de l'anneau s'étendent vers le haut en l'espèce trois picots 21 présentant la forme de tiges cylindriques dont l'extrémité supérieure est conique pour former une pointe dirigée vers le haut. Ces picots s'étendent de préférence à partir des trois régions de l'anneau correspondant aux trois « coins » du triangle arrondi formé par la cavité 11 du corps 10, et sont positionnés de manière à s'étendre sensiblement le long de la paroi intérieure définissant la partie haute 111 plus étroite de ladite cavité, de manière à réaliser également un guidage pour ladite translation verticale.

En particulier, les picots 21 sont ici disposés au ras du bord extérieur de l'anneau 22, tandis que la paroi 16 au niveau de la partie supérieure 111 de la cavité présente localement, au niveau des picots 21, de légers renfoncements 161 destinés à former des berceaux pour les picots respectifs en assurant le guidage précité.

On observera ici que l'ouverture centrale 221 de l'anneau permet, malgré la présence de l'armature 20, de conserver la continuité du passage entre la face supérieure et la face inférieure du corps 10, tel que défini par sa cavité 11.

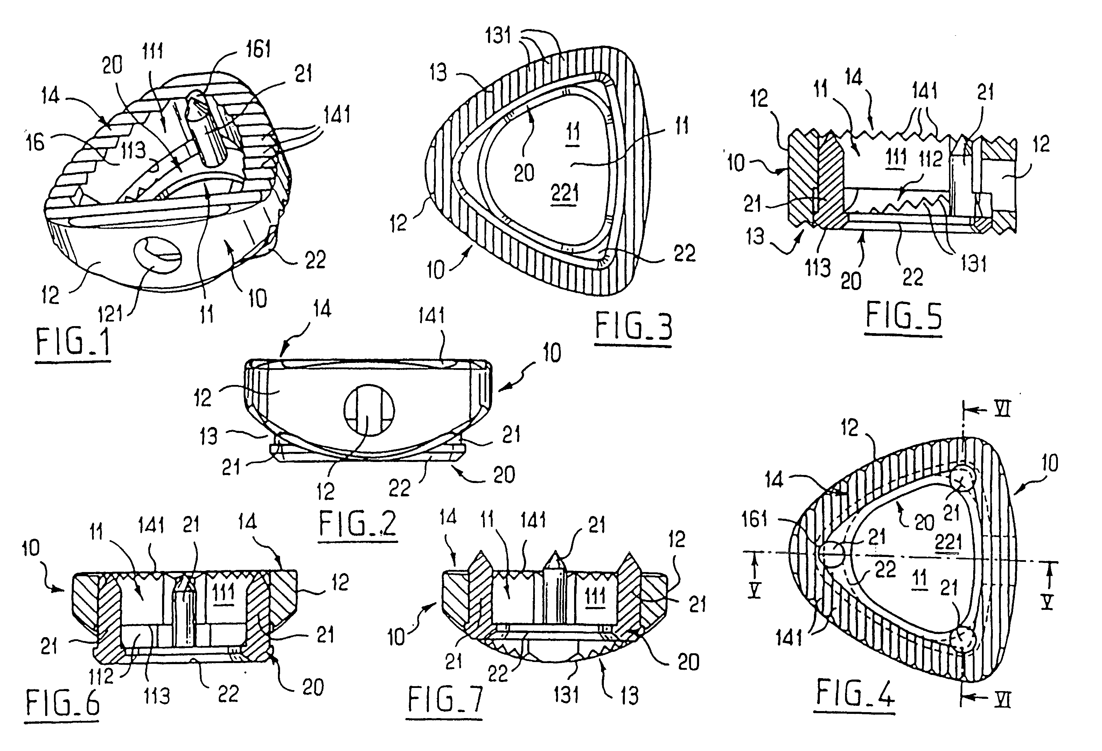

La pose de l'implant est effectuée en plaçant l'armature 20 dans le corps 10 dans la position illustrée sur les figures 2 et 6, c'est-à-dire de telle manière que les picots 21 soient légèrement en retrait ou affleurants par rapport à la face supérieure 14 du corps 10.

L'espace intérieur de l'implant défini par la cavité 11 est rempli en partie ou en totalité de greffon osseux ou d'une substance ostéogène. La distraction entre les plateaux vertébraux cervicaux étant effectuée, à l'aide d'une instrumentation classique en soi, l'implant est placé entre ces plateaux. En supprimant la distraction, l'implant se trouve comprimé entre les plateaux, et le plateau inférieur, dont on rappellera la concavité naturelle, sollicite vers le haut l'armature 20 en agissant au niveau de l'anneau 22, dont la planéité fait qu'au moment de la pose, certaines de ses régions débordent vers le bas par rapport à la face inférieure convexe 13 du corps. En exerçant cette pression, le plateau vertébral inférieur pousse l'armature 20 vers le haut, pour amener les picots 21 à déborder vers le haut au delà de la face supérieure 14 du corps (voir figure 7), et les extrémités en pointe de ces picots pénètrent dans le plateau vertébral supérieur pour ainsi assurer un ancrage particulièrement efficace. Les stries 131, 141 assurent quant à elles un ancrage secondaire, qui vient compléter l'ancrage principal réalisé par les picots 21.

On observera ici que la butée formée par l'épaulement 113 permet de limiter le mouvement de l'armature 20 vers le haut, et donc de bien contrôler le degré de pénétration des picots dans le plateau vertébral supérieur, sachant qu'une pénétration excessive risque de fragiliser le corps vertébral.

On observera également que dans cette position de butée, telle qu'illustrée sur la figure 7, l'anneau 22 de l'armature 20 est sensiblement dans une position d'affleurement par rapport à la face inférieure 13 du corps 10, si bien que l'appui du corps contre le plateau vertébral inférieur s'effectue dans de bonnes conditions, sur la surface maximale.

On comprend à la lecture de ce qui précède que l'armature 20, de par sa complémentarité de formes avec l'intérieur du corps 10, remplit une fonction de renforcement de ce corps. Il est ainsi particulièrement avantageux de réaliser l'armature 20 en un alliage métallique biocompatible tel qu'un alliage de titane ou un acier inoxydable, tandis que la corps 20 peut être réalisé en un polymère biocompatible, tel qu'un polyéther-éther-cétone (PEEK), sans que ne soit compromise la robustesse de l'implant.

On dispose ainsi d'un implant plus léger et plus facile et économique à fabriquer, sachant que le corps 10 peut alors être fabriqué par moulage.

En outre, le polymère étant radiotransparent, le fait que l'armature 20 soit métallique autorise la vérification post-chirurgicale de la tenue de l'implant par radiographie.

Ainsi l'armature 20 possède dans ce cas trois fonctions principales, à savoir une fonction d'ancrage principal, une fonction de renforcement du corps et une fonction de repérage radiographique.

Bien entendu, on peut jouer sur le nombre et la disposition des picots 21, de même que sur la taille et la forme de la cavité du corps 10 qui abrite l'armature. Il est toutefois préférable, pour assurer une fusion osseuse solide, que la section horizontale de la cavité 11 occupe au moins le quart de la section horizontale hors-tout de l'implant.

## Revendications

1. Implant formant une cage intersomatique, notamment pour vertèbres cervicales, comprenant un corps (10) possédant une face supérieure (14) et une face inférieure (13) aptes à s'appuyer sur les plateaux vertébraux sus- et sous-jacents et possédant en outre au moins une ouverture traversante (11) débouchant sur lesdites faces supérieure et inférieure, **caractérisé en ce qu'**il comprend en outre un organe d'ancrage (20) monté de façon à pouvoir se déplacer dans ladite ouverture, ledit organe d'ancrage comprenant une partie d'appui (22) apte à se trouver adjacente à un premier plateau vertébral, et au moins un élément saillant d'ancrage (21) apte à s'étendre à partir de ladite partie d'appui en direction du plateau vertébral opposé, l'implant tant apte à être comprimé entre les plateaux vertébraux de telle sorte que ledit organe d'ancrage (20) soit sollicité par le premier plateau vertébral au niveau de ladite partie d'appui pour se déplacer par rapport au corps et pour que ledit élément saillant (21) au moins prévu déborde de la face du corps tournée vers le plateau vertébral opposé et s'ancre dans ce dernier.

2. Implant selon la revendication 1, **caractérisé en ce que** le corps possède sur au moins l'une de ses faces supérieure et inférieure (14, 13) des aménagements d'ancrage complémentaire (141, 131).

3. Implant selon l'une des revendications 1 et 2, **caractérisé en ce que** ledit organe d'ancrage (20) possède une pluralité d'éléments saillants (21).

4. Implant selon la revendication 3, **caractérisé en ce que** lesdits éléments saillants (21) sont allongés et essentiellement parallèles entre eux.

5. Implant selon l'une des revendications 1 à 4, **caractérisé en ce que** le ou chaque élément saillant d'ancrage (21) présente la forme d'une tige à extrémité libre en pointe.

6. Implant selon l'une des revendications 1 à 5, **caractérisé en ce que** ladite ouverture (11) du corps possède un aménagement de butée (113) pour ledit organe d'ancrage (20), afin de limiter la course du déplacement de celui-ci.

7. Implant selon la revendication 6, **caractérisé en ce que** ledit aménagement est constitué par un épaulement (113) entre deux parties (111, 112) de tailles différentes de ladite ouverture (11).

8. Implant selon la revendication 7, **caractérisé en ce que** ladite partie d'appui (22) de l'organe d'ancrage s'adapte étroitement dans la partie de plus grande taille (112) de ladite ouverture (11).

9. Implant selon l'une des revendications 6 à 8, **caractérisé en ce que** lorsque l'organe d'ancrage s'appuie contre l'aménagement de butée (113), la partie d'appui (22) dudit organe est entièrement inscrite dans la face inférieure (13) du corps (10).

10. Implant selon l'une des revendications 7 à 9, **caractérisé en ce que** l'organe d'ancrage (20) possède une pluralité d'éléments saillants d'ancrage (21) s'adaptant ensemble étroitement dans la partie de plus petite taille (111) de ladite ouverture (11).

11. Implant selon l'une des revendications 1 à 10, **caractérisé en ce que** ladite partie d'appui (22) de l'organe d'ancrage (20) présente la forme d'un anneau dont une ouverture centrale (221) assure la continuité du passage entre les faces supérieure et inférieure du corps à travers ladite ouverture (11) du corps.

12. Implant selon l'une des revendications 1 à 11, **caractérisé en ce que** le corps (10) présente une forme extérieure généralement arrondie, avec des zones de forte courbure et des zones de courbure moindre.

13. Implant selon la revendication 12, **caractérisé en ce que** ladite ouverture (11) du corps présente un contour de forme semblable à ladite forme extérieure du corps.

14. Implant selon la revendication 13, **caractérisé en ce qu'**il est prévu sur l'organe d'ancrage (20) une pluralité d'éléments saillants (21) situés au voisinage de zones de forte courbure de ladite ouverture (11) et reçus dans des renfoncements (161) formés dans ces zones.

15. Implant selon l'une des revendications 1 à 14, **caractérisé en ce que** l'une (13) des faces supérieure et inférieure du corps (10) est convexe.

16. Implant selon la revendication 15, **caractérisé en ce que** la partie d'appui (22) de l'organe d'ancrage (20) est généralement plane.

17. Implant selon l'une des revendications 1 à 16, **caractérisé en ce que** le corps (10) est réalisé en un matériau polymère -biocompatible tel qu'un polyéther-éther-cétone.

18. Implant selon l'une des revendications 1 à 17, **caractérisé en ce que** l'organe d'ancrage (20) est réalisé en alliage métallique biocompatible.

## Patentansprüche

1. Implantat von der Art einer intersomatischen Höhle, insbesondere für Zervikalwirbel, das einen Körper (10) mit einer oberen Fläche (14) und einer unteren Fläche (13) aufweist, die sich an die oben und unten liegenden Wirbelflächen anpassen können, und der mindestens eine durchgehende Öffnung (11.) aufweist, die an diesen oberen und unteren Flächen mündet,
**dadurch gekennzeichnet, dass**
er außerdem ein Verankerungsorgan (20) aufweist, das so montiert ist, dass es sich in dieser durchgehenden Öffnung verschieben kam, wobei dieses Verankerungsorgan einen Auflageteil (22) enthält, der an eine erste Wirbelfläche angelegt werden kann, sowie mindestens ein hervorstehendes Verankerungselement (21), das sich ab dieser Auflagefläche in Richtung der gegenüberliegenden Wirbelfläche erstrecken kann, und das Implantat so zwischen den Wirbelflächen komprimiert werden kann, dass das Verankerungsorgan (20) mit Hilfe der ersten Wirbel fläche an dieser Auflagefläche gespannt wird, um sich gegenüber dem Körper zu verschieben, und dadurch mindestens eines der hervorstehenden Elemente (21) aus der Fläche des Körpers hervorsteht, die gegen die gegenüberliegende Wirbelfläche gerichtet ist, und in diesem verankert wird.

2. Implantat nach Anspruch 1,
**dadurch gekennzeichnet, dass**
der Körper mindestens an einer seiner oberen und unteren Flächen (14, 13) Einsätze für eine zusätzliche Verankerung (141,131) aufweist.

3. Implantat nach einem der Ansprüche 1 und 2,
**dadurch gekennzeichnet, dass**
dieses Verankerungsorgan (20) eine Vielzahl von hervorstehenden Elementen (21) aufweist.

4. Implantat nach Anspruch 3,
**dadurch gekennzeichnet, dass**
diese hervorstehenden Elemente (21) eine längliche Form haben und weitgehend parallel zueinander angeordnet sind.

5. Implantat nach einem der Ansprüche 1 bis 4,
**dadurch gekennzeichnet, dass**
das oder die hervorstehenden Verankerungselemente (21) die Form einer Spindel mit einem freien spitzen Ende haben.

6. Implantat nach einem der Ansprüche 1 bis 5,
**dadurch gekennzeichnet, dass**
die Öffnung (11) des Körpers einen Sitz (113) für das Verankerungselement (20) aufweist, um dessen Verschiebungshub zu begrenzen.

7. Implantat nach Anspruch 6,
**dadurch gekennzeichnet, dass**
dieser Sitz aus einer Schulter (113) besteht, die zwischen zwei Teilen (111, 112) unterschiedlicher Größe der Öffnung (11) angeordnet sind.

8. Implantat nach Anspruch 7,
**dadurch gekennzeichnet, dass**
der Auflageteil (22) des Verankerungsorgans im engen Sitz in dem größeren Teil (112) der Öffnung (11) angeordnet ist.

9. Implantat nach einem der Ansprüche 6 bis 8,
**dadurch gekennzeichnet, dass**
sich das Verankerungsorgan an dem Sitz (113) abstützt, und der Auflageteil (22) dieses Organs vollkommen von der unteren Fläche (13) des Körpers (10) eingefasst wird.

10. Implantat nach einem der Ansprüche 7 bis 9,
**dadurch gekennzeichnet, dass**
das Verankerungsorgan (20) eine Vielzahl von hervorstehenden Verankerungselementen (21) aufweist, die zusammen in engem Sitz in den kleineren Teil (111) der Öffnung (11) eingesetzt sind.

11. Implantat nach einem der Ansprüche 1 bis 10,
**dadurch gekennzeichnet, dass**
der Auflageteil (22) des Verankerungselementes (20) die Form eines Ringes hat, dessen Öffnung (221) den kontinuierlichen Durchgang zwischen den oberen und unteren Flächen des Körpers durch die Öffnung (11) des Körpers gewährleistet.

12. Implantat nach einem der Ansprüche 1 bis 11,
**dadurch gekennzeichnet, dass**
der Körper (10) eine weitgehend abgerundete Außenseite mit stark gekrümmten und weniger gekrümmten Bereichen aufweist.

13. Implantat nach Anspruch 12,
**dadurch gekennzeichnet, dass**
diese Öffnung des Körpers (11) ein Profil aufweist, das der äußeren Form des Körpers entspricht.

14. Implantat nach Anspruch 13,
**dadurch gekennzeichnet, dass**
auf dem Verankerungselement (20) eine Vielzahl von hervorstehenden Elementen (21) vorgesehen ist, die im Bereich starker Krümmungen der Öffnung (11) vorgesehen sind, und in Verstärkungen (161) eingesetzt sind, die in diesen Bereichen ausgebildet sind.

15. Implantat nach einem der Ansprüche 1 bis 14,
**dadurch gekennzeichnet, dass**
eine (13) der oberen und unteren Flächen des Körpers (10) eine konvexe Form hat.

16. Implantat nach Anspruch 15,
**dadurch gekennzeichnet, dass**
der Auflageteil (22) des Verankerungsteils (20) weitgehend flach ausgebildet ist.

17. Implantat nach einem der Ansprüche 1 bis 16,
**dadurch gekennzeichnet, dass**
der Körper (10) aus einem biologisch abbaubaren Polymermaterial besteht, wie zum Beispiel einem Polyetherätherketon.

18. Implantat nach einem der Ansprüche 1 bis 17,
**dadurch gekennzeichnet, dass**
das Verankerungselement (20) aus einer biologisch abbaubaren Legierung besteht.

## Claims

1. Implant forming an interbody cage, particularly for cervical vertebrae, comprising a body (10) which has an upper face (14) and a lower face (13), which are capable of bearing on the overlying and underlying vertebral plates, and also comprising at least one through-opening (11) which opens out on said upper and lower faces, **characterized in that** it additionally comprises an anchoring member (20) mounted in such a way as to be able to move in said opening, said anchoring member comprising a bearing portion (22) which can be located adjacent to a first vertebral plate, and at least one projecting anchoring element (21) which is capable of extending from said bearing portion in the direction of the opposite vertebral plate, the implant being capable of being compressed between the vertebral plates such that said anchoring member (20) is biased by the first vertebral plate in the area of said bearing portion so as to move in relation to the body and so that said at least one projecting element (21) protrudes from that face of the body facing the opposite vertebral plate and anchors in the latter.

2. Implant as claimed in Claim 1, **characterized in that** the body has, on at least one of its upper and lower faces (14, 13), complementary anchoring arrangements (141, 131).

3. Implant as claimed in either of Claims 1 and 2, **characterized in that** said anchoring member (20) has a plurality of projecting elements (21).

4. Implant as claimed in Claim 3, **characterized in that** said projecting elements (21) are elongate and essentially parallel to one another.

5. Implant as claimed in any of Claims 1 to 4, **characterized in that** the projecting anchoring element or each projecting anchoring element (21) has the form of a rod with a pointed free end.

6. Implant as claimed in any of Claims 1 to 5; **characterized in that** said opening (11) of the body has a bearing arrangement (113) for said anchoring member (20) in order to limit the range of movement of the latter.

7. Implant as claimed in Claim 6, **characterized in that** said arrangement consists of a shoulder (113) between two parts (111, 112) of different sizes of the said opening (11).

8. Implant as claimed in Claim 7, **characterized in that** said bearing portion (22) of the anchoring member fits tightly into the larger-sized part (112) of said opening (11).

9. Implant as claimed in any of Claims 6 to 8, **characterized in that** when the anchoring member bears against the bearing arrangement (113), the bearing portion (22) of said member is entirely inscribed within the lower face (13) of the body (10).

10. Implant as claimed in any of Claims 7 to 9, **characterized in that** the anchoring member (20) has a plurality of projecting anchoring elements (21) all fitting tightly into the smaller-sized part (111) of said opening (11).

11. Implant as claimed in any of Claims 1 to 10, **characterized in that** said bearing portion (22) of the anchoring member (20) has the form of a ring in which a central opening (221) ensures continuity of passage between the upper and lower faces of the body, through said opening (11) of the body.

12. Implant as claimed in any of Claims 1 to 11, **characterized in that** the body (10) has a generally rounded external form, with zones of greater curvature and zones of lesser curvature.

13. Implant as claimed in Claim 12, **characterized in that** said opening (11) of the body has a contour with a form similar to said outer form of the body.

14. Implant as claimed in Claim 13, **characterized in that** the anchoring member (20) is provided with a plurality of projecting elements (21) situated in the vicinity of zones of greater curvature of said opening (11) and received in recesses (161) formed in these zones.

15. Implant as claimed in any of Claims 1 to 14, **characterized in that** one (13) of the upper and lower faces of the body (10) is convex.

16. Implant as claimed in Claim 15, **characterized in that** the bearing portion (22) of the anchoring member (20) is generally plane.

17. Implant as claimed in any of Claims 1 to 16, **characterized in that** the body (10) is made of a biocompatible polymer material such as polyetherether ketone.

18. Implant as claimed in any of Claims 1 to 17, **characterized in that** the anchoring member (20) is made of a biocompatible metal alloy.
